# EUROPEAN PATENT SPECIFICATION

(11) **EP 3 893 713 B1**
(45) Date of publication and mention of the grant of the patent: **15.04.2026**
(21) Application number: 19842436.8
(22) Date of filing: 11.12.2019
(51) Int. Cl.: A61B 1/00, A61B 1/307

(54) **URETEROSCOPE DEVICES, SYSTEMS, AND METHODS**
URETEROSKOPE, SYSTEME UND METHODEN
DISPOSITIFS D'URÉTÉROSCOPE, SYSTÈMES ET MÉTHODES

(30) Priority: 13.12.2018 US 201862779263 P
(43) Date of publication of application: 20.10.2021
(60) Divisional application: 26164156.7
(73) Proprietor: C. R. Bard, Inc., Franklin Lakes, NJ 07417 (US)
(72) Inventor: KNOLLMAN, Kevin Michael, West Chester, Ohio 45069 (US); WESNER, Kyra Kormos, Loveland, Ohio 45140 (US); LEWIS, Emily Lorraine, Decatur, Georgia 30033 (US); HUDNALL, Janet Kim, Atlanta, Georgia 30308 (US)
(74) Representative: Hoffmann Eitle
(86) International application number: PCT/US2019/065616
(87) International publication number: WO 2020/123588

(56) References cited:
- EP-A1- 3 048 946
- WO-A1-2016/049490
- WO-A1-2017/192351
- JP-B2- 3 898 993
- US-A- 5 307 803
- US-A1- 2013 190 561
- US-A1- 2014 200 402
- US-A1- 2015 208 909
- US-A1- 2016 174 819
- US-A1- 2016 213 387

## Description

### BACKGROUND

Endoscopes of small size are desired in many industrial and medical applications. For example, when natural orifices and lumens of a human body are small, small endoscopes are used for insertion through such orifices and lumens to target locations within the body. For single incision laparoscopy, smaller endoscopes are preferred to provide an inside-the-body view of the surgical site, particularly when the incision itself is of minimal dimensions. Sometimes, patients may feel irritation when an endoscope is being inserted into his or her body, and a smaller endoscope may mitigate such unpleasant experience and may minimize trauma to the patient. Moreover, a physician may improve diagnostic and procedural protocols with a smaller endoscope. For example, transnasal endoscopy may sometimes replace trans-oral endoscopy.

WO 2017/192351 discloses an insertion device that is within a uteroscope that has a handle designed to be held comfortably in the hand. At the back end of the handle it is disclosed to place there an actuator for wires to steer the leading end of a shaft of the insertion device.

WO 2016/049490 and US 2013/0190561 each disclose a steerable medical device that can be used for uteroscopy. It features a handle and at the back end of the handle is provided an actuator for steering the device.

US 2016/0213387 discloses a retrieval apparatus with a handle which includes a steering mechanism to deflect the distal end of the device and a control mechanism to translate a retrieval device with a working chamber.

WO 2015/047631 discloses an endoscope with multiple viewing elements. It features a handpiece equipped with buttons or knobs or switches to control bending of the endoscope shaft, as well as such functions as fluid injection and suction.

US 2014/0200402 discloses a medical device introduction system with an introducer body that supports a position controller in the form of a wheel to be held in the hand of a user and has grooves around its circumference to assist manipulation of a working channel of the introducer system by a user.

US 2016/0174819 discloses a battery-powered handpiece with a central generally cylindrical portion on which is carried a power ON/OFF button for a display screen at the end of the handpiece remote from the endoscope shaft.

### SUMMARY

The invention is defined in the independent claim below. The dependent claims are directed to optional features and preferred embodiments. Embodiments disclosed herein are related to ureteroscope systems and the disclosure includes methods of operating ureteroscope systems. In an embodiment, a ureteroscope system is disclosed. The ureteroscope system includes a handpiece, a catheter, and a cable. The handpiece has a catheter end, a control end opposite to the catheter end, a working channel port positioned proximate to the catheter end and distal to the control end, and a cable port positioned proximate to the catheter end and distal to the control end. The catheter extends from the catheter end of the handpiece. The catheter includes a working channel in fluid communication with the working channel port, a distal end, and an image sensor positioned at the distal end of the catheter. The cable is connected or connectable to the handpiece at the cable port and configured to provide power to the image sensor and communicate with one or more electronic devices.

The handpiece has a catheter end, a control end opposite to the catheter end, central portion, and a working channel port. The central portion is positioned between the catheter end and the control end. The central portion has a rounded first surface, a rounded second surface opposite to the rounded first surface, and two opposing substantially flat sides between the rounded first surface and the rounded second surface. A longitudinal central plane of the handpiece is positioned between the two opposing substantially flat sides. The working channel port is disposed on the rounded first surface along the longitudinal central plane proximate to the catheter end and distal to the control end. The catheter extends from the catheter end of the handpiece. The catheter includes a working channel in fluid communication with the working channel port, a distal end, and an image sensor positioned at the distal end of the catheter.

The handpiece can include a communication interface configured to communicate with at least one of one or more electronic devices, and the one or more controls positioned on the handpiece proximate to the control end. The one or more electronic devices include at least a display operable to display one or more images collected by the image sensor. At least one of the one or more controls can be configured to adjust at least one of a brightness, a zoom, a focus, or a contrast of the one or more images displayed on the display.

The invention enables a method of operating a ureteroscope system that includes a step of inserting the catheter of the ureteroscope into a patient. The method includes operably coupling a ureteroscope to one or more electronic devices, the one or more electronic devices including at least a display and the ureteroscope including handpiece and a catheter connected to a catheter end of the handpiece. The catheter includes a distal end and an image sensor positioned at the distal end of the catheter. The method also includes displaying one or more images collected by the image sensor on the display. The method also includes adjusting at least one of a brightness, a zoom, a focus, or a contrast of the one or more images displayed on the display using at least one of one or more controls positioned on a control end of the handpiece distal to the catheter end.

In addition, other features and advantages of the present disclosure will become apparent to those of ordinary skill in the art through consideration of the following detailed description and the accompanying drawings.

### BRIEF DESCRIPTION OF THE DRAWINGS

The drawings illustrate several embodiments of the present invention, wherein identical reference numerals refer to identical or similar elements or features in different views or embodiments shown in the drawings.
**FIG. 1A** illustrates a digital ureteroscope as part of an endoscope system.
**FIG. 1B** illustrates an end view of a distal end of the catheter of the ureteroscope of **FIG. 1A****.**
**FIG. 2** is a side view of the handpiece of the ureteroscope of **FIG. 1A** with a side of the handpiece removed
**FIG. 3** is a side view of the handpiece of the ureteroscope of **FIG. 1A****.**
**FIG. 4** is a catheter end view of the handpiece of the ureteroscope of **FIG. 1A****.**
**FIG. 5** is a control end view of the handpiece of the ureteroscope of **FIG. 1A****.**
**FIG. 6** is a top view of the handpiece of the ureteroscope of **FIG. 1A****.**
**FIG. 7** is a bottom view of the handpiece of the ureteroscope of **FIG. 1A****.**
**FIG. 8** is an isometric view of the handpiece of the ureteroscope of **FIG. 1A****.**
**FIG. 9** is a flow diagram of a method of operating a ureteroscope system.

### DETAILED DESCRIPTION

Embodiments disclosed herein relate to devices and systems, and enable methods of using an endoscope, such as a ureteroscope. The endoscope systems include a handpiece and a catheter. Conventional endoscopes may sometimes be difficult or inefficient to hold or maneuver during use. For example, handpieces of conventional endoscopes are typically not designed for use with either the left hand or the right hand. In an embodiment of a ureteroscope system described herein, the ureteroscope may be used by either the right hand or the left hand, and also may be switch from hand to hand during use. The ureteroscope system includes a handpiece and a catheter. The handpiece includes a catheter end, a control end opposite to the catheter end, a central portion, and a working channel port. The central portion is positioned between the catheter end and the control end. The central portion has a rounded first surface, a rounded second surface opposite to the rounded first surface, and two opposing substantially flat sides between the rounded first surface and the rounded second surface. A longitudinal central plane of the handpiece is positioned between the two opposing substantially flat sides. The working channel port is disposed on the rounded first surface along the longitudinal central plane proximate to the catheter end and distal to the control end. The catheter extends from the catheter end of the handpiece and includes a working channel in fluid communication with the working channel port, a distal end, and an image sensor positioned at the distal end of the catheter. Positioning of the working channel port in line with or along the central plane of the handpiece enables the ureteroscope to be used with either a left or a right hand, or switched between hands during use.

Maneuverability of the handpiece during use also is improved by location of the working port channel and/or a cable connected to the handpiece. In an embodiment, a ureteroscope system includes a handpiece, a catheter, and a cable. The handpiece has a catheter end, a control end opposite to the catheter end, a working channel port positioned proximate to the catheter end and distal to the control end, and a cable port positioned proximate to the catheter end and distal to the control end. The catheter extends from the catheter end of the handpiece. The catheter includes a working channel in fluid communication with the working channel port, a distal end, and an image sensor positioned at the distal end of the catheter. The cable is connected to the handpiece at the cable port and is configured to provide power to the image sensor and communicate with one or more electronic devices. Moreover, with both the working port channel and the cable connected to the handpiece positioned proximate to the catheter end of the handpiece, the working port channel and the cable are between a hand holding the handpiece and the catheter. Positioning of both the cable port and the working channel port proximate to the catheter end of the handpiece rather than distal to the catheter end improves ergonomics and maneuverability of the handpiece during use.

According to the invention, the endoscopes described herein also include one or more controls positioned on the handpiece that improve the functionality and use of the endoscope. According to the invention, a ureteroscope system includes a catheter and a handpiece. The catheter includes a distal end and an image sensor positioned at the distal end of the catheter. The handpiece has a catheter end, a control end opposite to the catheter end, a communication interface configured to communicate with at least one of one or more electronic devices, and one or more controls positioned on the handpiece proximate to the control end. The one or more electronic devices include at least a display operable to display one or more images collected by the image sensor and at least one of the one or more controls is configured to adjust at least one of a brightness, a zoom, a focus, or a contrast of the one or more images displayed on the display. The one or more controls on the endoscope allow a user to adjust views presented on the display and/or computer according to the user's preference and as necessary during use of the ureteroscope. The handpiece includes at least one control configured to activate an optoelectronic module, for switching image modes of the optoelectronic module, and for activating a frame grabber to create a still image from the video stream output generated by the optoelectronic module. The handpiece includes a light activation control on the handpiece that is configured to turn on or off a light at the distal end of the catheter. In some instances of use, it is desirable to turn the light of the endoscope completely off during backloading of the endoscope over guidewires, rather than merely incrementally adjusting the light.

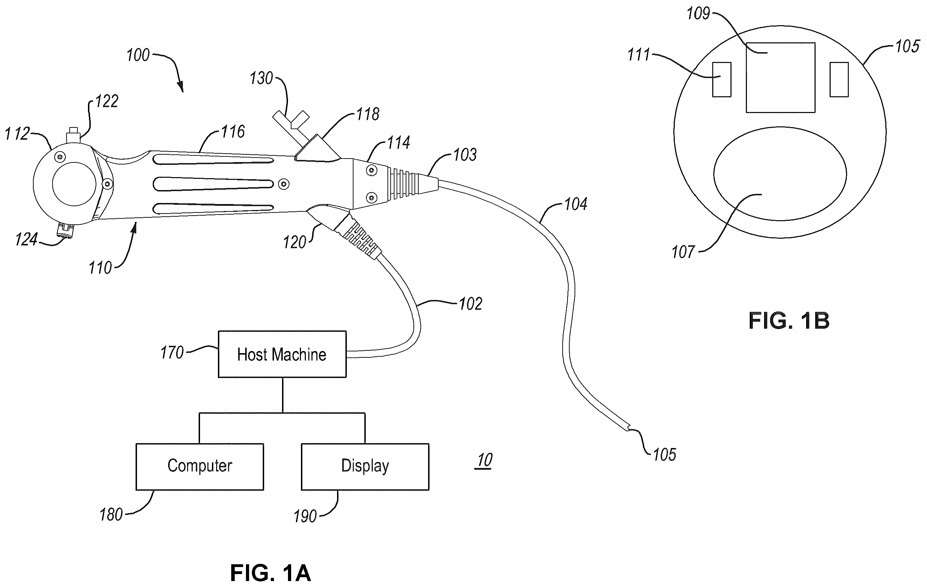

**FIG. 1A** illustrates an endoscopic system 10. In an embodiment, the endoscopic system 10 includes a digital ureteroscope 100 operably coupled to one or more electronic devices, such as a host machine 170 and one or more external terminals including a display 190 and a computer 180. The ureteroscope 100 may be operably coupled to one or more of the host machine 170, the computer 180, or the display 190 wirelessly or through a cable 102. The ureteroscope 100 includes a catheter 104 and a handpiece 110 having a catheter end 114 and a control end 112 opposite or distal to the catheter end 114. As the control end 112 is opposite to the catheter end 114, the control end 112 and the catheter end 114 are at different ends of the handpiece 110 and may face different directions relative to one another. In an embodiment, both the catheter 104 and the handpiece 110 are disposable. In some embodiments, the catheter 104 and the handpiece 110 are manufactured as an integral part, or the catheter 104 is fixed with the handpiece 110 via a handpiece-catheter connector 103. Alternatively, only the catheter 104 is disposable, and the handpiece 110 may be sterilized and reused multiple times. In this case, the catheter 104 is removably connected to the handpiece 110 and the catheter end 114 via a handpiece-catheter connector 103.

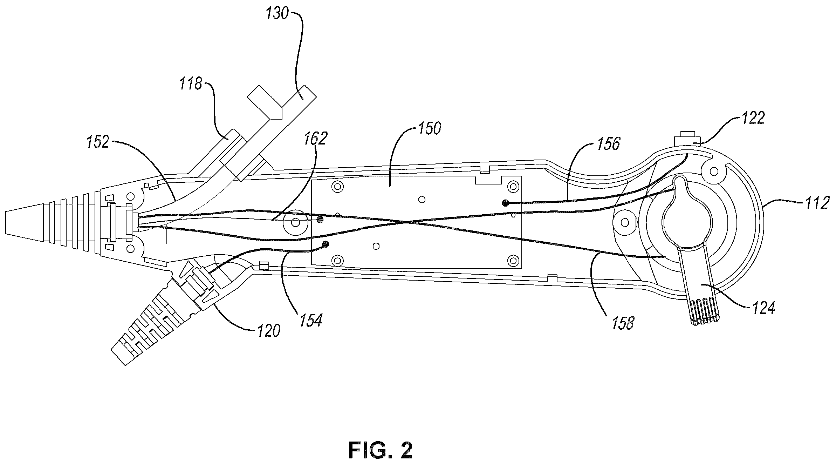

The catheter 104 of the ureteroscope 100 may be used for imaging an interior surface of a tubular structure, such as a lumen in the body of human or animal. For example, the catheter 104 may be inserted via a subject's urethra to access various parts of the urinary tract. However, it should be appreciated that the ureteroscope 100 may be employed as an industrial endoscope when tubular structure is a part of an industrial apparatus, an equipment, a product, a machine, a production line, and the like. In some embodiments, the catheter 104 may serve as a tether, and may include a plurality of scale markings or fiducials that enable a physician to measure a distance traveled by optoelectronic module into the tubular structure, such as a lumen of a body. Other structure(s) may be built into the ureteroscope 100 as desired. The handpiece 110 includes a steering controller 124 configured to control one or more steering wires 158 **(****FIG. 2****)** that are connected to an active bend portion of the catheter 104 to deflect the distal end 105 to the desired location. Accordingly, a user may bend or curve the catheter 102 by moving the steering controller 124 on the handpiece 110.

The ureteroscope 100 includes an optoelectronic module (e.g., a camera or other imager) for imaging the interior of the subject. For example, turning to **FIG. 1B****,** an optoelectronic module 109 and at least one light source 111 may be located in a distal end 105 of the catheter 104 or other location in the catheter 104. The optoelectronic module 109 may include a micro camera module having an image sensor microchip, a set of micro lenses, and a micro illumination module. Suitable optoelectronic modules are disclosed in U.S. Patent No. 9,942,452. In some embodiments, the optoelectronic module 109 may be positioned in a rigid or semi-rigid shell-like housing at the distal end 105 configured for insertion into the tubular structure for imaging its interior surface. For example, the optoelectronic module 109 may be inserted into a patient's body through a natural body orifice, such as the mouth, nose, urethra, bladder, vagina, or anus. The ureteroscope 100 may therefore have different configurations for use as a gastrointestinal, a colonoscope, endoscopic ultrasound (EUS), endoscopic retrograde cholangiopancreatography (ERCP), or other suitable application. Applications of the ureteroscope 100 include diagnostic observation associated with endometrial polyps, infertility, abnormal bleeding, and pelvic pain, and surgical procedure such as embryo growth arrest and uterine malformation etc.

The ureteroscope 100 may include a receiving device or communication interface 150 **(****FIG. 2****)** generally located outside the catheter 104 for receiving the signal from an image sensor within the optoelectronic module 109. For example, the catheter 104 may include at least one electrical lead 162 **(****FIG. 2****)** that is coupled to the optoelectronic module 109 and conveys an electrical signal from optoelectronic module 109 to the communication interface 150. The communication interface 150 may be positioned within or adjacent to the handpiece 110.

The catheter 104 may be configured to couple the optoelectronic module 109 to the circuitry within the handpiece 110 in any suitable manner. For example, the availability of low-cost modular imaging system components enables the manufacture of a disposable components of the ureteroscope 100 at very low cost. In an embodiment, the catheter 104 is configured to detachably couple the optoelectronic module 109 to circuitry within the handpiece 110. In this manner, the catheter 104 and the optoelectronic module 109 are disposable, and may be detached from the handpiece 110 after a single patient use, thus eliminating the need for sterilization or reprocessing and reducing contamination risks. The handpiece 110 may be disinfected for subsequent reuse with a catheter 104 and optoelectronic module 109 for a different patient.

Returning to **FIG. 1A****,** the endoscopic system 10 may include one or more electronic devices for processing and displaying the image data received from the optoelectronic module 109 of the ureteroscope 100. For example, the endoscopic system 10 may include one or more of a host machine 170 having a microprocessor, a computer 180 having a microprocessor, and a display 190. The host machine 170 may be connected to one or more terminals of the computer 180 and the display 190 for further processing and displaying the image data from the optoelectronic module 109. The host machine 170 or the computer 180 may be programmed with image processing software that takes as input the image data output from the optoelectronic module 109 of the ureteroscope 100 and generates two- or three-dimensional reconstructions of the body lumen that may be displayed on the display 190. Accordingly, a processor in at least one of the host machine 170, the computer 180, or the display 190 may be programmed with software that accepts as input a plurality of still images of an object generated by the optoelectronic module 109, and then output for display a three-dimensional rendering of the object based on the plurality of still images. The display 190 may include any suitable display, and may be configured to display a moving image (movie) or a still image collected by the image sensor of the optoelectronic module 109. Although shown in **FIG. 1A** as separate blocks, the host machine 170, the computer 180, and the display 190 may include a single device, two devices, three devices, or more than three devices.

The endoscopic system also includes a cable 102 configured to operably couple the ureteroscope to at least one of the host machine 170, the computer 180, or the display 190. The cable 102 may electrically couple the retrieving device 150 **(****FIG. 2****)** of the ureteroscope 100 to at least one of the host machine 170, the computer 180, or the display 190. The cable 102 also allows the communication interface 150 to communicate with and receive electric power from the host machine 170 or other power sources. The cable 102 also may be configured to allow the communication interface 150 to transmit image data captured at the optoelectronic module 109 to the host machine 170 for processing, storing, and displaying.

Turning to **FIG. 2****,** which illustrates a side view of the handpiece 110 with a portion of the handpiece 110, the ureteroscope 100 may include a communication interface 150 or host interface housed in the handpiece 110. The communication interface 150 may contain, for example, one or more of a processor board, a camera board and frame grabber, or a power source. The processor board may be coupled by the cable 102 to the host machine 170 for storage and retrieval of images generated by ureteroscope 100. The communication interface 150 also may be configured to communicate with and receive electric power from the host machine 170 or other power source via the cable 102. The communication interface 150 also may transmit image data captured at the distal end 105 to the host machine 170 for processing, storing, and displaying. The communication interface 150 may be connected to the cable 102 through one or more wires 154 and/or connected to the optoelectronic module 109 through one or more electrical leads 162.

Alternatively or in addition, the communication interface 150 or handpiece 110 may include an antenna and a wireless chipset, e.g., compliant with the IEEE 802.11 WiFi standards, for wirelessly transmitting the video or still image generated by the ureteroscope 100 to the host machine 170, the computer 180, or the display 190 without the cable 102. For example, the communication interface 150 may include a wireless interface configured to implement various protocols, including but not limited to Wi-Fi, Bluetooth, ZigBee, Z-Wave, etc. This arrangement may be useful in a physician's office because it permits the computer and display to be placed outside of the sterile field, while also allowing the physician greater maneuverability during use of the ureteroscope 100. In other embodiments, the communication interface 150 may be omitted and the image data may be transmitted directly to the host machine 170 and/or the computer 180.

The ureteroscope 100 includes one or more controls 122 positioned at or proximate to the control end 112 of the handpiece 110. The one or more controls 122 may include one or more of a switch, a button, a rotatable knob, a movable tab, and the like. The one or more controls may be electrically coupled to the communication interface 150 through one or more wires 156. In some embodiments, the one or more controls 122 are configured to adjust views presented on the display 190. For example, the one or more controls may be configured to adjust at least one of a brightness, a zoom, a focus or a contrast of one or more images displayed on the display 190. Accordingly, the one or more controls 122 allow a user to adjust views presented on the display 190 and/or computer 180 according to the user's preference and as necessary during use of the ureteroscope 100.

Adjustment of at least one of the brightness, the zoom, the focus, or the contrast of the one or more images displayed on the display 190 may result from at least one of the one or more controls 122 adjusting at least one of the brightness, the zoom, the focus, or the contrast on the optoelectronic module 109. Adjustment of at least one of the brightness, the zoom, the focus, or the contrast of the one or more images displayed on the display 190 may result from at least one of the one or more controls 122 adjusting at least one of the brightness, the zoom, the focus, or the contrast on the display 190. In some embodiments, at least one of the one or more controls 122 is configured to allow a user to switch between adjusting at least one of the brightness, the zoom, the focus, or the contrast on the optoelectronic module 109 and adjusting at least one of the brightness, the zoom, the focus, or the contrast on the display 190.

The ureteroscope 100 may include one or more wires coupling the one or more controls 122 to the optoelectronic module 109 to allow a user to adjust at least one of the brightness, the zoom, the focus, or the contrast on the optoelectronic module 109. For example, at least one of the one or more controls 122 may be connected to the optoelectronic module 109 through one or more wires 156 connected to the communication interface 150 and at least one of the one or more control 122 and an electric lead 162 connected to the communication interface 150 and the optoelectronic module 109. In some embodiments, one or more wires or electric leads may be connected directly to the optoelectronic module 109 and at least one of the one or more control 122. Coupling the one or more controls 122 to the communication interface 150 may allow a user to adjust at least one of the brightness, the zoom, the focus, or the contrast on the display 190, either wirelessly or through the cable 102.

In some embodiments, a processor of the host machine 170 or another processor coupled to the ureteroscope 100 is configured to display one or more view settings on the display 190. The one or more view settings may display responsive actuation of the one or more controls 122 or, alternatively, an additional button on the handpiece 110 or display 190. The one or more view settings displayed on the display 190 and/or computer 180 may include at least one of brightness, zoom, contrast, or focus. For example, responsive to actuation of at least one of the one or more controls 122, a brightness setting may be displayed on the display 190. After the brightness setting is displayed on the display 190, a user may adjust the brightness of the one or more images displayed on the display 190 using at least one of the one or more controls. Other view settings may be similarly displayed on the display 190 and adjusted by the user with the one or more controls 122. In some embodiments, the one or more view settings also may be displayed on a processor display on the handpiece 110 or the host machine 170.

At least one of the one or more controls 122 is configured to activate (e.g., turn on) or deactivate (e.g., turn off) at least one light source 111 **(****FIG. 1B****)** at the distal end 105 of the catheter 104. For example, the light source 111 may include a light-emitting diode (LED) positioned in the distal end 105 of the catheter. Alternatively or in addition, the ureteroscope 100 may include a LED light source positioned elsewhere, such as in the handpiece 110, which provides illumination to the at least one light source 111 at the distal end 105 via one or more sets of optical fibers extending through the catheter 104. In some instances of use, it is desirable to turn the at least one light source 111 of the ureteroscope 100 completely off during backloading of the ureteroscope 100 over guidewires, rather than merely incrementally adjusting the light. Accordingly, at least one of the one or more controls allows a user to selectively turn off and turn on the light source 111 when desired by the user.

At least one of the one or more controls 122 is configured to activate and deactivate the optoelectronic module 109. At least one of the one or more controls 122 also is configured to switch an image mode in at least one of the optoelectronic module 109, the retrieving device 150, or the one or more electronic devices between a still image mode whereby a still image is recorded and a video image mode whereby a video stream is recorded. At least one of the one or more controls 122 also is configured to activate a frame grabber mode in at least one of the optoelectronic module 109, the retrieving device 150, or the one or more electronic devices that creates a still image from the video stream output generated by the optoelectronic module 109. For example, at least one of the one or more controls 122 may be configured to communicate with one or more of the host machine 170, the computer 180, or the display 190 to allow a user to create a still image from a video stream being displayed on the display 190, and storing or recording the still image on at least one of the host machine 170 or the computer 180.

The ureteroscope 100 may be operated to perform or complete selected tasks manually, automatically, or a combination thereof. Some ureteroscopic functions may be implemented with the use of components that comprise hardware, software, firmware or combinations thereof. While general-purpose components such as general purpose computers or oscilloscopes may be used in the ureteroscope 100, dedicated or custom components such as circuits, integrated circuits or software may be too. For example, some functions are implemented with a plurality of software instructions executed by one or more data processors, which is part of a general-purpose or custom computer. The one or more data processors may be in at least one of the communication interface 150, the host machine 170, the computer 180, or the display 190. In some embodiments, the data processor or computer comprises volatile memory for storing instructions and/or data and/or a non-volatile storage, for example, a magnetic hard-disk and/or removable media, for storing instructions and/or data. In some embodiments, implementation includes a network connection. In some embodiments, implementation includes a user interface, generally comprising one or more input devices (e.g., allowing input of commands and/or parameters) and output devices (e.g., allowing reporting parameters of operation and results).

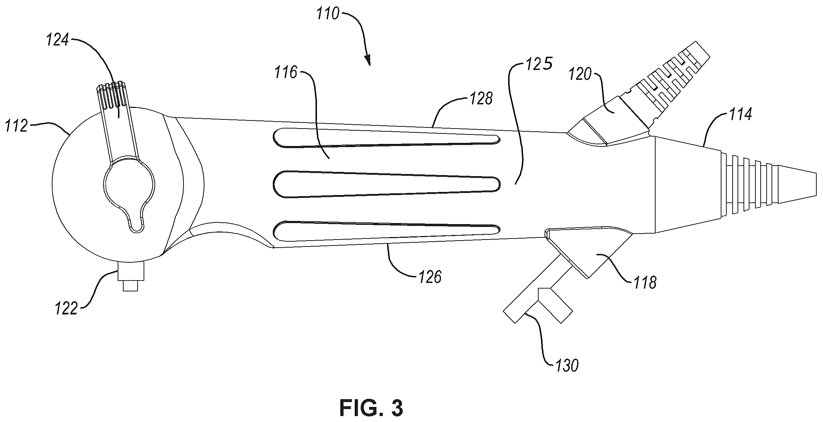

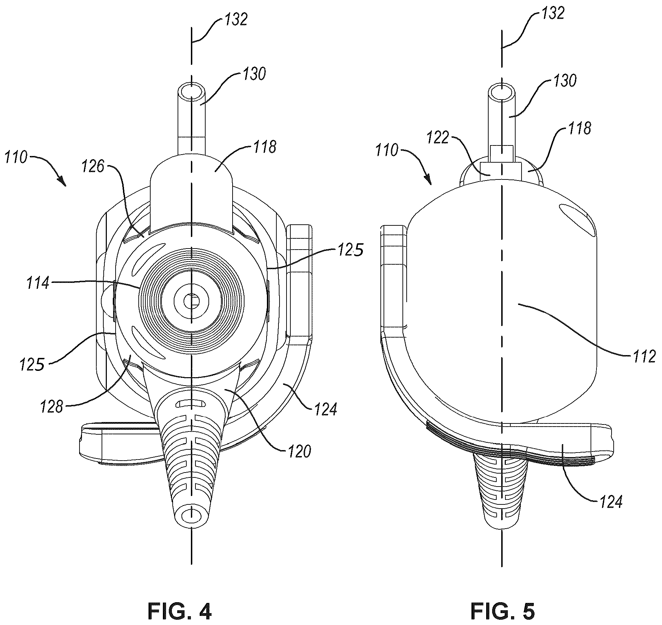

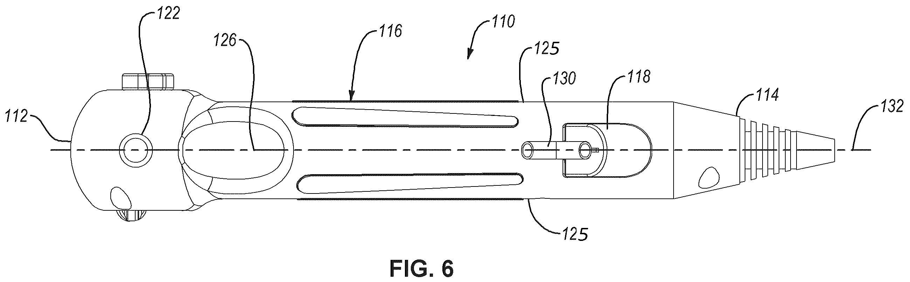

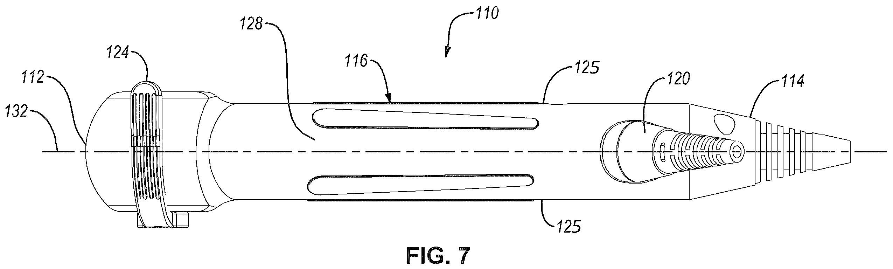

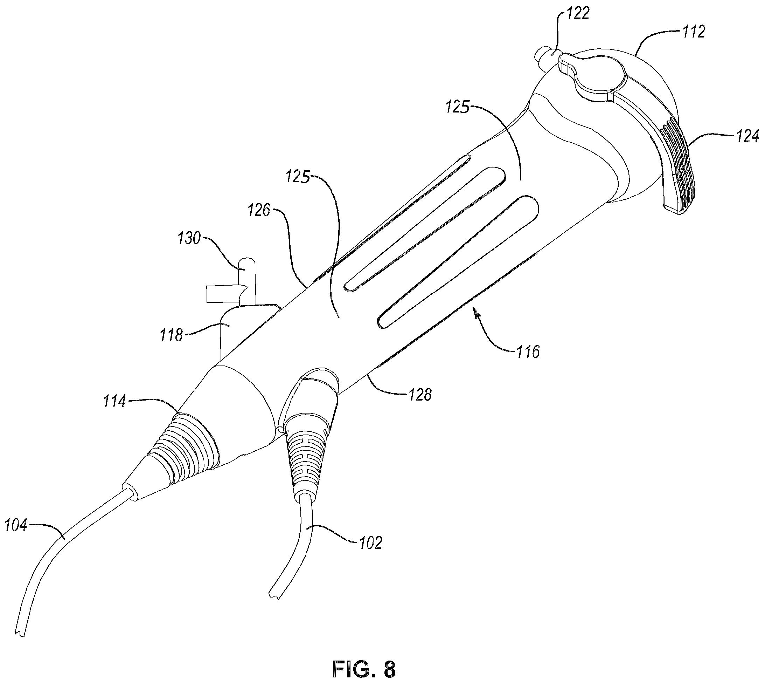

The handpiece 110 of the ureteroscope 100 also is configured to improve maneuverability and efficiency of use. **FIG. 3** is a side view of the handpiece 110, **FIG. 4** is a catheter end 114 view of the handpiece 110, **FIG. 5** is a control end 112 view of the handpiece 110, **FIG. 6** is a top view of the handpiece 110, **FIG. 7** is a bottom view of the handpiece 110, and **FIG. 8** is an isometric view of the handpiece 110. References to **FIGS. 3-8** are made below. The handpiece 110 is elongated and includes the catheter end 114 proximate to the catheter and the control end 112 opposite or distal to the catheter end 114. The control end 112 may include a generally bulbous shape, and the handpiece 110 may taper between the control end 112 and the catheter end 114.

With the control end 112 opposite or distal to the catheter end 114, an intermediate or central portion 116 of the handpiece 110 is positioned between the catheter end 114 and the control end 112. The central portion 110 includes a rounded first or top surface 126, a rounded second or bottom surface 128, and two opposing substantially flat sides 125 positioned between the top surface 126 and the bottom surface 128. A first substantially flat side of the two opposing substantially flat sides 125 may face a first direction, and a second substantially flat side of the two opposing substantially flat side 125 may face a second direction that is different or away from the first direction of the first substantially flat side. A theoretical or imaginary longitudinal central plane 132 **(****FIGS. 4-6****)** may divide the central portion 116 substantially in half. For example, the longitudinal central plane 132 may extend through an approximate center of the rounded top surface 126 and an approximate center of the rounded bottom surface 128, with the longitudinal central plane 132 thereby extending through a central axis of the handpiece 110 and being positioned approximately halfway between the two opposing substantially flat sides 125.

The handpiece 110 also includes a working channel port 118 and a working channel 107 **(****FIG. 1B****)** that provides fluid communication between the working channel port 118 and the distal end 105 of the catheter 104. The handpiece 110 also may include a working channel connector 130 that is connected or attached to the working channel port 118 and a conduit 152 **(****FIG. 2****)** that provides fluid communication between working channel port 118 and the working channel 107 through the handpiece 110. At least one of the working channel port 118 or the working channel connector 130 is configured to engage with various surgical instruments and irrigation devices, as needed, for operations such as stone breaking and retrieval, etc. As shown in **FIGS. 4-6****,** the working channel port 118 is positioned generally in line with the longitudinal central plane 132. For example, the working channel port 118 extends from rounded top surface 126 generally along the longitudinal central plane 132. As such, the working channel port 118 may be positioned on the rounded top surface 126 to be centered on the longitudinal central plane 132. Positioning of the working channel port 118 generally in line with the longitudinal central plane 132 of the handpiece 110 enables the handpiece 110 to be used with either a left or a right hand, or switched between hands during use.

The working channel port 118 also may be positioned on a lower or bottom portion of the handpiece 110, proximate to the catheter end 114 of the handpiece 110. For example, the working channel port 118 may be positioned less than one-half of a distance from the catheter end 114 to the control end 112, less than one-third of the distance from the catheter end 114 to the control end 112, less than one-quarter of the distance from the catheter end 114 to the control end 112, or less than one-fifth of the distance from the catheter end 114 to control end 112.

The handpiece 110 also includes a cable port 120 for receiving or connecting the cable 102 **(****FIGS. 1A** and **8****),** thereby operably coupling the ureteroscope 100 to at least one of the host machine 170, the computer 180, or the display 190. As shown in **FIGS. 4-6****,** the cable port 120 is positioned generally in line with the longitudinal central plane 132. For example, the cable port 120 extends from the rounded bottom surface 128 generally along the longitudinal central plane 132. As such, the cable port 120 is positioned on the rounded bottom surface 128 to be centered on the longitudinal central plane 132. Positioning of the cable port 120 generally in line with the longitudinal central plane 132 of the handpiece 110 enables the handpiece 110 to be used with either a left or a right hand, or switched between hands during use.

Both the cable port 120 and the working channel port 118 are positioned on a lower or bottom portion of the handpiece 110, proximate to the catheter end 114 of the handpiece 110. For example, both the cable port 120 and the working channel port 118 may be positioned less than one-half of a distance from the catheter end 114 to the control end 112, less than one-third of the distance from the catheter end 114 to the control end 112, less than one-quarter of the distance from the catheter end 114 to the control end 112, or less than one-fifth of the distance from the catheter end 114 to control end 112. Connecting the cable 102 to the cable port 120 positioned on the lower or bottom portion of the handpiece 110 improves the ergonomics of the handpiece 110. For example, the cable 102 is less likely to interfere with use of the ureteroscope 100 when the cable 102 is positioned on the lower or bottom portion of the handpiece 110, as opposed to the conventional position of a cable at an end of the handpiece opposite or distal to the catheter.

Alternatively, the handpiece 110 may further include a compact battery module for supplying power to the optoelectronic module 109 and the at least one light source 111. The power source in the handpiece 110 may be, for example, one or more conventional dry-cell disposable batteries or lithium ion rechargeable batteries.

Moreover, the ureteroscope 100 shown in **FIGS. 1-8** includes a cable port 120 positioned proximate to the catheter end 114 and distal to the control end 112. The handpiece 110 includes one or more controls 122 positioned at the control end 112 of the handpiece 110 and a working channel port 118 disposed on the rounded first surface 126 along the longitudinal central plane 132 proximate to the catheter end 114. The handpiece includes a working channel port 118 disposed on the rounded first surface 126 along the longitudinal central plane 132 proximate to the catheter end 114.

The cable port 120 is positioned proximate to the catheter end 114.

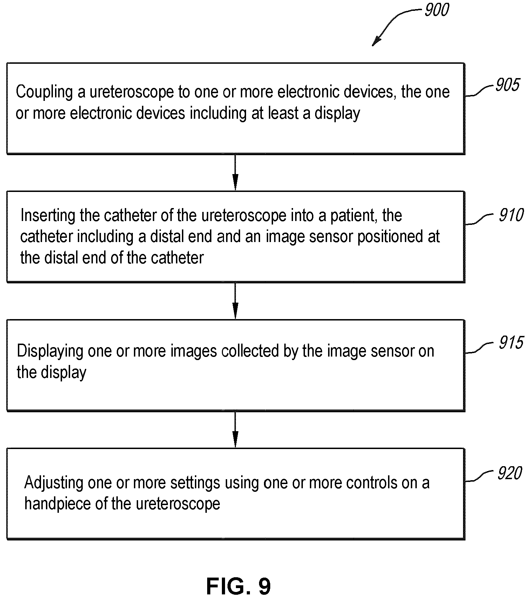

**FIG. 9** is a flow diagram of a method 900 of operating a ureteroscope system. The method, which is not covered by the claimed invention, includes an act 905 of coupling a ureteroscope to one or more electronic devices. The method also includes an act 910 of inserting the catheter of the ureteroscope into a patient. The method also includes an act 915 of displaying one or more images collected by the image sensor of the ureteroscope on the display. The method also includes an act 920 of adjusting one or more settings using one or more controls on a handpiece of the ureteroscope.

Acts 905, 910, 915, and 920 of the method 900 are for illustrative purposes. For example, the acts 905, 910, 915, and 920 of the method 900 may be performed in different orders, split into multiple acts, modified, supplemented, or combined. In an embodiment, one or more of the acts 905, 910, 915, and 920 of the method 900 may be omitted from the method 900. Any of the acts 905, 910, 915, and 920 of the method 900 may include using any of the handpieces 110, ureteroscopes 100, or the system 10 disclosed herein.

The act 905 of coupling a ureteroscope to one or more electronic devices may include operably coupling a ureteroscope to one or more electronic devices, the one or more electronic devices including at least a display and the ureteroscope including handpiece and a catheter connected to a catheter end of the handpiece. In some embodiments, operably coupling a ureteroscope to one or more electronic devices may include connecting a cable of the ureteroscope to at least one of the one or more electronic devices, the cable being connected to the handpieces of the ureteroscope. In some embodiments, operably coupling a ureteroscope to one or more electronic devices includes wirelessly coupling a retrieving device of the handpiece to at least one of the one or more electronic devices.

The act 910 of inserting the catheter of the ureteroscope into a patient may include inserting the distal tip of the catheter into a lumen in the body of human or animal. For example, the catheter may be inserted via a subject's urethra to access various parts of the urinary tract.

The act 915 of displaying one or more images collected by the image sensor of the ureteroscope on the display may include displaying at least one of a still image or a video stream on the display using the one or more images collected by the image sensor. The act 915 also may include displaying two- or three-dimensional reconstructions of the body lumen on the display. The two- or three-dimensional reconstructions of the body lumen may be generated using at least one of a host machine or a computer programmed with image processing software that takes as input the image data output from the image sensor of the ureteroscope.

The act 920 of adjusting one or more settings using one or more controls on a handpiece of the ureteroscope may include adjusting at least one of a brightness, a zoom, a focus, or a contrast of the one or more images displayed on the display using at least one of one or more controls positioned on a control end of the handpiece distal to the catheter end. The act 920 of adjusting one or more settings using one or more controls on a handpiece of the ureteroscope may include activating or deactivating the image sensor using at least one of the one or more controls. The act 920 of adjusting one or more settings using one or more controls on a handpiece of the ureteroscope may include activating at least one light source at the distal end of the catheter using at least one of the one or more controls. The act 920 of adjusting one or more settings using one or more controls on a handpiece of the ureteroscope may include changing an image collection mode of the image sensor from a still image mode to a video stream mode using the one or more controls. The act 920 of adjusting one or more settings using one or more controls on a handpiece of the ureteroscope may include activating a frame grab to record a still image during the video stream mode using at least one of the one or more controls.

Acts 905, 910, 915, and 920 of the method 900 are for illustrative purposes. For example, the acts 905, 910, 915, and 920 of the method 900 may be performed in different orders, split into multiple acts, modified, supplemented, or combined. In an embodiment, one or more of the acts 905, 910, 915, and 920 of the method 900 may be omitted from the method 900. Any of the acts 905, 910, 915, and 920 of the method 900 may include using any of the handpieces 110, ureteroscopes 100, or the system 10 disclosed herein.

As used herein, the term "about" or "substantially" refers to an allowable variance of the term modified by "about" or "substantially" by ±10% or ±5%. Further, the terms "less than," "or less," "greater than," "more than," or "or more" include, as an endpoint, the value that is modified by the terms "less than," "or less," "greater than," "more than," or "or more."

The various embodiments disclosed herein are for purposes of illustration.

## Claims

1. A ureteroscope (100) system, comprising:
a catheter having a distal end (105) in which is at least one light source (111) and an optoelectronic module (109) for imaging the interior of a subject and for outputting a video stream; and
a handpiece (110) including:
a catheter end (114);
a control end (112) opposite to the catheter end;
a central portion (116) positioned between the catheter end and the control end, the central portion having a rounded first surface (126), a rounded second surface (128) opposite to the rounded first surface, and two opposing substantially flat sides (125) between the rounded first surface and the rounded second surface, a longitudinal central plane (132) of the handpiece being positioned between the two opposing substantially flat sides; and
a working channel port (118) disposed on the rounded first surface along the longitudinal central plane proximate to the catheter end and distal to the control end; and
a cable port (120) disposed on the rounded second surface along the longitudinal central plane proximate to the catheter end and distal to the control end; and
a cable (102) connected or connectable to the handpiece at the cable port and configured to provide power to an image sensor in the optoelectronic module and communicate with one or more electronic devices and a communication interface;
the catheter (104) extending from the catheter end of the handpiece, the catheter including an active bend portion and a working channel (107) in fluid communication with the working channel port, the ureteroscope system further comprising one or more controls (122) positioned on the handpiece proximate to the control end,
wherein one or more first controls of the one or more controls is configured to activate and deactivate the at least one light source (111) and one or more second controls of the one or more controls, different from the one or more first controls, is configured to activate the optoelectronic module, for switching image modes of the optoelectronic module, for activating a frame grabber to create a still image from the video stream output generated by the optoelectronic module; and
wherein the handpiece includes a steering controller (124) configured to control one or more steering wires (158) that are connected to the active bend portion of the catheter to deflect the distal end (105) to the desired location; and
wherein the working channel port (118), the cable port (120), the controls (122) and the steering controller (124) are positioned generally in line with the longitudinal central plane (132) of the handpiece (110) thereby to enable the handpiece (110) to be used by either the right hand or the left hand or switched between hands during use.

2. The ureteroscope system of claim 1, wherein the handpiece includes a communication interface (150) configured to communicate with one or more electronic devices.

3. The ureteroscope system of claim 2, wherein the one or more electronic devices include at least a display (190) operable to display one or more images collected by the image sensor.

4. The ureteroscope system of claim 3, wherein one or more third controls of the one or more controls, different from the one or more first controls and the one or more second controls, is configured to adjust at least one of a brightness, a zoom, a focus, or a contrast of the one or more images displayed on the display.

5. The ureteroscope system of any one of claims 3 or 4, further comprising the one or more electronic devices including a host machine (170).

6. The ureteroscope system of claim 5, wherein the host machine is configured to display one or more view settings on the display responsive to actuation of at least one of the one or more controls, the one or more view settings including at least one of the brightness, the zoom, the focus, or the contrast of the one or more images displayed on the display.

## Patentansprüche

1. Ureteroskop- (100) -System, umfassend:
einen Katheter, der ein distales Ende (105) aufweist, in welchem mindestens eine Lichtquelle (111) und ein optoelektronisches Modul (109) zum Abbilden des Inneren eines Subjekts und zum Ausgeben eines Video-Streams angeordnet sind; und
ein Handstück (110), einschließend:
ein Katheterende (114);
ein Steuerende (112) gegenüber dem Katheterende;
einen zentralen Abschnitt (116), der zwischen dem Katheterende und dem Steuerende positioniert ist, wobei der zentrale Abschnitt eine abgerundete erste Oberfläche (126), eine abgerundete zweite Oberfläche (128) gegenüber der abgerundeten ersten Oberfläche und zwei gegenüberliegende im Wesentlichen flache Seiten (125) zwischen der abgerundeten ersten Oberfläche und der abgerundeten zweiten Oberfläche aufweist, wobei eine longitudinale Mittelebene (132) des Handstücks zwischen den zwei gegenüberliegenden im Wesentlichen flachen Seiten positioniert ist; und
einen Arbeitskanalanschluss (118), der auf der abgerundeten ersten Oberfläche entlang der longitudinalen Mittelebene in der Nähe des Katheterendes und distal zum Steuerende angeordnet ist; und
einen Kabelanschluss (120), der auf der abgerundeten zweiten Oberfläche entlang der longitudinalen Mittelebene in der Nähe des Katheterendes und distal zum Steuerende angeordnet ist; und
ein Kabel (102), das mit dem Handstück am Kabelanschluss verbunden oder verbindbar ist und dazu konfiguriert ist, einem Bildsensor in dem optoelektronischen Modul Leistung bereitzustellen und mit einem oder mehreren elektronischen Vorrichtungen und einer Kommunikationsschnittstelle zu kommunizieren;
den Katheter (104), der sich von dem Katheterende des Handstücks erstreckt, wobei der Katheter einen aktiven Biegeabschnitt und einen Arbeitskanal (107) in Fluidverbindung mit dem Arbeitskanalanschluss einschließt, wobei das Ureteroskopsystem weiter ein oder mehrere Bedienelemente (122) umfasst, die auf dem Handstück in der Nähe des Steuerendes positioniert sind,
wobei ein oder mehrere erste Bedienelemente der einen oder mehreren Bedienelemente dazu konfiguriert sind, die mindestens eine Lichtquelle (111) zu aktivieren und zu deaktivieren, und ein oder mehrere zweite Bedienelemente der einen oder mehreren Bedienelemente, die verschieden von den einen oder mehreren ersten Bedienelementen sind, dazu konfiguriert sind, das optoelektronische Modul zu aktivieren, um Bildmodi des optoelektronischen Moduls umzuschalten, um einen Framegrabber zu aktivieren, um ein Standbild aus der von dem optoelektronischen Modul erzeugten Video-Stream-Ausgabe zu erstellen; und
wobei das Handstück eine Lenksteuereinheit (124) einschließt, die dazu konfiguriert ist, einen oder mehrere Lenkdrähte (158) zu steuern, die mit dem aktiven Biegeabschnitt des Katheters verbunden sind, um das distale Ende (105) zu dem gewünschten Ort auszulenken; und
wobei der Arbeitskanalanschluss (118), der Kabelanschluss (120), die Bedienelemente (122) und die Lenksteuereinheit (124) im Allgemeinen in einer Linie mit der longitudinalen Mittelebene (132) des Handstücks (110) positioniert sind, um dadurch zu ermöglichen, dass das Handstück (110) entweder von der rechten Hand oder der linken Hand verwendet oder während der Verwendung zwischen den Händen gewechselt werden kann.

2. Ureteroskopsystem nach Anspruch 1, wobei das Handstück eine Kommunikationsschnittstelle (150) einschließt, die dazu konfiguriert ist, mit einem oder mehreren elektronischen Vorrichtungen zu kommunizieren.

3. Ureteroskopsystem nach Anspruch 2, wobei die eine oder mehrere elektronische Vorrichtungen mindestens eine Anzeige (190) einschließen, die betriebsfähig ist, ein oder mehrere von dem Bildsensor gesammelte Bilder anzuzeigen.

4. Ureteroskopsystem nach Anspruch 3, wobei ein oder mehrere dritte Bedienelemente der einen oder mehreren Bedienelemente, die verschieden von den einen oder mehreren ersten Bedienelementen und den einen oder mehreren zweiten Bedienelementen sind, dazu konfiguriert sind, mindestens eine von einer Helligkeit, einem Zoom, einem Fokus oder einem Kontrast der auf der Anzeige angezeigten einen oder mehreren Bilder einzustellen.

5. Ureteroskopsystem nach einem der Ansprüche 3 oder 4, weiter umfassend die eine oder mehrere elektronische Vorrichtungen, die eine Hostmaschine (170) einschließen.

6. Ureteroskopsystem nach Anspruch 5, wobei die Hostmaschine dazu konfiguriert ist, eine oder mehrere Ansichtseinstellungen auf der Anzeige als Reaktion auf eine Betätigung mindestens eines der einen oder mehreren Bedienelemente anzuzeigen, wobei die eine oder mehrere Ansichtseinstellungen mindestens eines der Merkmale Helligkeit, Zoom, Fokus oder Kontrast der auf der Anzeige angezeigten einen oder mehreren Bilder einschließen.

## Revendications

1. Système d'urétéroscope (100), comprenant :
un cathéter présentant une extrémité distale (105) dans laquelle sont présents au moins une source lumineuse (111) et un module optoélectronique (109) pour imager l'intérieur d'un sujet et pour émettre en sortie un flux vidéo ; et
une pièce à main (110) incluant :
une extrémité de cathéter (114) ;
une extrémité de commande (112) opposée à l'extrémité de cathéter ;
une partie centrale (116) positionnée entre l'extrémité de cathéter et l'extrémité de commande, la partie centrale présentant une première surface arrondie (126), une deuxième surface arrondie (128) opposée à la première surface arrondie, et deux côtés opposés sensiblement plats (125) entre la première surface arrondie et la deuxième surface arrondie, un plan central longitudinal (132) de la pièce à main étant positionné entre les deux côtés opposés sensiblement plats ; et
un port de canal de travail (118) disposé sur la première surface arrondie le long du plan central longitudinal à proximité de l'extrémité de cathéter et en position distale par rapport à l'extrémité de commande ; et
un port de câble (120) disposé sur la deuxième surface arrondie le long du plan central longitudinal à proximité de l'extrémité de cathéter et en position distale par rapport à l'extrémité de commande ; et
un câble (102) relié ou pouvant être relié à la pièce à main au niveau du port de câble et configuré pour fournir une alimentation à un capteur d'image dans le module optoélectronique et communiquer avec un ou plusieurs dispositifs électroniques et une interface de communication ;
le cathéter (104) s'étendant depuis l'extrémité de cathéter de la pièce à main, le cathéter incluant une partie de courbure active et un canal de travail (107) en communication fluidique avec le port de canal de travail, le système d'urétéroscope comprenant en outre une ou plusieurs commandes (122) positionnées sur la pièce à main à proximité de l'extrémité de commande,
dans lequel une ou plusieurs premières commandes parmi les une ou plusieurs commandes sont configurées pour activer et désactiver au moins une source lumineuse (111) et une ou plusieurs deuxièmes commandes parmi les une ou plusieurs commandes, différentes des une ou plusieurs premières commandes, sont configurées pour activer le module optoélectronique, pour
commuter des modes d'image du module optoélectronique, pour activer un captureur d'image pour créer une image fixe à partir du flux vidéo de sortie généré par le module optoélectronique ; et
dans lequel la pièce à main inclut un dispositif de commande de guidage (124) configuré pour commander un ou plusieurs fils de guidage (158) qui sont reliés à la partie de courbure active du cathéter pour dévier l'extrémité distale (105) vers l'emplacement souhaité ; et
dans lequel le port de canal de travail (118), le port de câble (120), les commandes (122) et le dispositif de commande de guidage (124) sont positionnés généralement en ligne avec le plan central longitudinal (132) de la pièce à main (110), ce qui permet à la pièce à main (110) d'être utilisée soit par la main droite, soit par la main gauche, ou d'être échangée entre les mains durant l'utilisation.

2. Système d'urétéroscope selon la revendication 1, dans lequel la pièce à main inclut une interface de communication (150) configurée pour communiquer avec un ou plusieurs dispositifs électroniques.

3. Système d'urétéroscope selon la revendication 2, dans lequel les un ou plusieurs dispositifs électroniques incluent au moins une unité d'affichage (190) apte à afficher une ou plusieurs images collectées par le capteur d'image.

4. Système d'urétéroscope selon la revendication 3, dans lequel une ou plusieurs troisièmes commandes parmi les une ou plusieurs commandes, différentes des une ou plusieurs premières commandes et des une ou plusieurs deuxièmes commandes, sont configurées pour ajuster au moins l'un parmi la luminosité, le zoom, la mise au point ou le contraste d'une ou plusieurs images affichées sur l'unité d'affichage.

5. Système d'urétéroscope selon l'une quelconque des revendications 3 ou 4, comprenant en outre les un ou plusieurs dispositifs électroniques incluant une machine hôte (170).

6. Système d'urétéroscope selon la revendication 5, dans lequel la machine hôte est configurée pour afficher un ou plusieurs réglages de vue sur l'unité d'affichage en réponse à l'actionnement d'au moins une commande parmi les une ou plusieurs commandes, les un ou plusieurs réglages de vue incluant au moins l'un parmi la luminosité, le zoom, la mise au point ou le contraste d'une ou plusieurs images affichées sur l'unité d'affichage.
